# FASCICULE DE BREVET EUROPEEN

(11) **EP 0 614 656 B1**
(45) Date de publication et mention de la délivrance du brevet: **09.10.1996**
(21) Numéro de dépôt: 94400184.1
(22) Date de dépôt: 28.01.1994
(51) Int. Cl.: A61K 7/00, A61K 9/06

(54) **Gels aqueux contenant des microsphères creuses expansées**
Wässrige Gele, die expandierte hohle Mikrosphären enthalten
Aqueous gels containing expanded hollow microspheres

(30) Priorité: 29.01.1993 FR 9300990
(43) Date de publication de la demande: 14.09.1994
(73) Titulaire: L'OREAL, F-75008 Paris (FR)
(72) Inventeur: Bara, Isabelle, F-75013 Paris (FR); Mellul, Myriam, F-94240 L'Hay Les Roses (FR)
(74) Mandataire: Tonnellier, Jean-Claude

(56) Documents cités:
- EP-A- 0 486 394
- DE-A- 1 569 467
- GB-A- 2 238 242

## Description

L'invention a pour objet de nouvelles compositions cosmétiques ou dermopharmaceutiques sous forme de gels aqueux modifiés par addition de microsphères expansées.

On sait que dans la préparation de compositions cosmétiques ou dermopharmaceutiques, on utilise souvent des compositions sous forme d'émulsions, telles que des crèmes. Les émulsions, qui peuvent être du type eau-dans-l'huile ou huile-dans-l'eau, sont constituées de deux phases non miscibles et d'un tensio-actif dont le rôle est de stabiliser la dispersion de l'une des phases, ou phase dispersée, dans la phase continue.

De telles émulsions sont généralement onctueuses. Toutefois, en fonction de la quantité et de la qualité d'huile employée, l'utilisateur peut ressentir un effet gras plus ou moins désagréable, voire un effet collant. En outre, l'émulsionnant, dont la présence est nécessaire pour stabiliser l'émulsion, peut s'avérer plus ou moins irritant pour la peau.

Par ailleurs, certaines compositions cosmétiques ou dermopharmaceutiques sont présentées sous forme de gels aqueux. On sait qu'un gel est un état intermédiaire entre l'état solide et l'état liquide.

On admet qu'un gel est constitué par un réseau tridimensionnel de molécules qui retient dans ses mailles une quantité importante de solvant. La formation d'un tel réseau constitue la gélification. Selon la nature du solvant, on obtient des hydrogels ou organogels.

Un des intérêts de l'utilisation de compositions sous forme d'hydrogels est que ceux-ci apportent une agréable sensation de fraîcheur à l'application, sans effet gras déplaisant. Toutefois, il apparaît un effet collant et un effet de tension de la peau, pendant une application avec massage, ou après l'application, lorsque l'eau s'évapore.

On a maintenant découvert que, de façon surprenante, il est possible d'obtenir des compositions conservant les qualités des gels, sans en avoir les inconvénients, grâce à l'introduction de microsphères creuses expansées dans un gel hydrophile sans huile. Malgré l'absence d'huile, les gels modifiés ainsi préparés présentent une onctuosité durable lors de l'application, qui est particulièrement facile et agréable. Même après séchage, la peau n'a aucun toucher collant et l'utilisateur ne ressent aucune sensation de tension de la peau.

Jusqu'à présent, les microsphères creuses expansées avaient été utilisées dans des compositions sous forme de poudres, ou sous forme d'émulsions, ou encore pour obtenir des compositions ayant l'aspect d'une mousse ; voir par exemple les brevets JP-60 184004, FR-2.472.382, FR-2.658.720, FR-2.669.222 ou DE-2.521.003. En pratique, les compositions cosmétiques connues utilisant des microsphères creuses expansées contenaient toujours des matières grasses. Il n'était pas évident que l'incorporation de telles microsphères dans des gels aqueux, sans huile, permettrait d'obtenir une onctuosité lors de l'étalement qui n'était obtenue jusqu'à présent qu'avec les compositions contenant des matières grasses.

Ainsi, les compositions de l'invention apportent beaucoup de confort et de douceur à l'application. En outre, après application, la peau conserve un aspect mat. Par ailleurs, les compositions de l'invention ont une viscosité qui reste sensiblement constante lorsque la température est élevée (contrairement à ce qu'on observe couramment avec les crèmes) de sorte que leur utilisation reste agréable même dans les pays chauds ou pendant les saisons chaudes.

L'invention a donc pour objet une composition cosmétique ou dermopharmaceutique, ou un support de composition cosmétique ou dermopharmaceutique, sous forme de gel modifié, caractérisé(e) par le fait que ladite composition ou ledit support comprend des microsphères creuses dispersées dans un gel aqueux, que lesdites microsphères creuses sont des microsphères expansées, en matériau thermoplastique ayant une masse spécifique de 15 à 200 kg/m3, que ladite composition ou ledit support contient de 0,1 à 10% en poids desdites microsphères par rapport au poids total de la composition ou du support, et que ladite composition ou ledit support est exempt(e) de matières grasses.

Les microsphères expansées en matériau thermoplastique sont connues et peuvent être obtenues par exemple selon les procédés décrits dans les brevets et demandes de brevet EP-56219, EP-348372, EP-486080, EP-320473, EP-112807 et US-3.615.972.

Ces microsphères peuvent être réalisées en tous matériaux thermoplastiques non toxiques et non irritants. On peut utiliser par exemple des polymères ou copolymères d'acrylonitrile ou de chlorure de vinylidène. On peut utiliser par exemple un copolymère contenant, en poids, de 0 à 60% de motifs dérivés de chlorure de vinylidène, de 20 à 90% de motifs dérivés d'acrylonitrile et de 0 à 50% de motifs dérivés d'un monomère acrylique ou styrènique, la somme des pourcentages (en poids) étant égale à 100. Le monomère acrylique est par exemple un acrylate ou méthacrylate de méthyle ou d'éthyle. Le monomère styrènique est par exemple l'alpha-méthyl-styrène ou le styrène. Ces microsphères peuvent se présenter à l'état sec ou hydraté.

La cavité interne des microsphères creuses expansées contient un gaz, qui peut être un hydrocarbure tel que l'isobutane, l'isopentane, ou encore de l'air. Parmi les microsphères creuses utilisables, on citera en particulier celles commercialisées sous la marque Expancel par la société Kémanord Plast, en particulier de qualité DE (état sec) ou WE (état hydraté).

Dans les compositions ou supports de compositions de l'invention, le pourcentage pondéral des microsphères creuses expansées est de préférence compris entre 0,5 et 5%. La texture des gels est particulièrement onctueuse pour des pourcentages de 0,5 à 2%, généralement de 1 à 2% environ. La texture est plus pâteuse pour des proportions de 2 à 5% qui conviennent plus particulièrement pour certains produits de nettoyage de la peau ou pour des produits destinés à être appliqués en couche épaisse (masques).

Les microsphères creuses ont généralement des dimensions moyennes de particules pouvant varier de 5 à 250µm, et en particulier de 10 à 150µm.

Pour réaliser le gel qui est à la base de la composition ou du support de l'invention, on utilise au moins un agent gélifiant dans un véhicule liquide aqueux. Bien entendu, l'agent gélifiant est présent en quantité suffisante pour conférer à la composition la viscosité souhaitée, qui est évidemment fonction de l'utilisation envisagée. Cette viscosité peut aller par exemple de 3 à 200 poises (soit 0,3 à 20 Pa.s).

Par exemple, lorsqu'on souhaite obtenir des produits onctueux, on ajuste la viscosité à une valeur comprise entre 10 et 50 poises (soit entre 1 et 5 Pa.s). Lorsqu'on souhaite réaliser des produits plus consistants, utilisables notamment comme masques, on ajuste la viscosité par exemple entre 20 et 200 poises et en particulier entre 20 et 100 poises. Pour des compositions utilisées comme produits de gommage pour la peau (scrubs), la viscosité est comprise entre notamment 10 et 100 poises environ (soit entre 1 et 10 Pa.s).

Les agents gélifiants sont choisis notamment parmi les polymères hydrosolubles ou donnant, dans l'eau, des solutions colloïdales.

Ce sont notamment les polymères ou copolymères d'acides organiques carboxyliques insaturés ou d'esters insaturés, les dérivés polysaccharidiques, les gommes, les silicates colloïdaux, les polyéthylèneglycols (PEG) et leurs dérivés, les polyvinylpyrrolidones et leurs dérivés, les polyacrylamides et leurs dérivés, les polyacrylonitriles et les gels de silice hydrophiles.

Les agents gélifiants sont par exemple les polymères ou copolymères acryliques et/ou méthacryliques, les polymères carboxyvinyliques, les acrylates ou méthacrylates de polyglycéryle, les dérivés de polyacrylamides tels que le Sepigel 305 (Seppic), le PAS 5161 ou le Bozepol C (Hoechst), les polyacrylonitriles comme l'Hypan SS 201 (Kingston/Lipo Chemical Inc., les dérivés de cellulose ou d'amidon, les dérivés de chitine, les alginates, l'acide hyaluronique et ses sels, les chondroïtine sulfates, les gommes de xanthane, de gellane, de Rhamsan, de karoya, ou de guar, la farine de caroube, et les silicates d'aluminium et de magnésium colloïdaux de type montmorillonite.

On peut citer notamment comme agents gélifiants particuliers : les polymères carboxyvinyliques vendus sous le dénomination Carbopol (Goodrich), les copolymères acide acrylique/acrylate d'éthyle, les copolymères acide acrylique/méthacrylate de stéaryle, le polyglycérylméthacrylate vendu sous la dénomination Lubrajel (Guardian), le polyglycérylacrylate vendu sous la dénomination Hispagel (Hispano Chimica), la carboxyméthylcellulose, l'hydroxyméthylcellulose, l'hydroxypropylcellulose, la cellulose microcristalline, l'hydroxypropylguar, les hectorites ou bentonites colloïdales vendues sous la dénomination Veegum, etc...

Les compositions de l'invention peuvent aussi contenir les divers ingrédients utilisés dans les compositions cosmétiques ou dermopharmaceutiques, notamment des pigments, des colorants, des agents conservateurs, des agents hydratants, des parfums, des agents de texture tels que des agents pulvérulents autres que des microsphères creuses, des agents absorbant l'ultraviolet, etc...

Les pigments peuvent être des pigments minéraux, des pigments organiques ou des pigments nacrés. Parmi les pigments minéraux, on peut citer par exemple le dioxyde de titane, les oxydes de fer noir, jaune, rouge ou brun, le violet de manganèse, le violet d'outre-mer, le bleu d'outre-mer, l'oxyde de chrome, etc...

Parmi les pigments organiques on peut citer en particulier les pigments D & C Red n°3, n°6, n°7, n°9, n°13, n°19, n°21, n°27, n°30 ou n°36, ou encore le noir de carbone.

Les pigments nacrés peuvent être choisis parmi les pigments nacrés blancs, tel que le mica recouvert d'oxyde de titane ou d'oxychlorure de bismuth. On peut également utiliser des pigments nacrés colorés, tel que le mica titane coloré avec des oxydes de fer ou avec de l'oxyde de chrome, le mica titane coloré avec un pigment organique du type précité, ou encore des pigments nacrés à base d'oxychlorure de bismuth.

Comme colorants, on peut utiliser des colorants hydrosolubles tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, etc...

Parmi les ingrédients solubles dans l'eau utilisables, on peut citer en particulier les polyols tel que le propylèneglycol, le butylène-1,3 glycol, la glycérine, la polyglycérine, le sorbitol, le glucose, le saccharose, le gluconate de magnésium, les oligo-élements, les acides silicones solubles dans l'eau, etc...

Certaines compositions de l'invention (notamment des produits de maquillage ou de nettoyage) peuvent également contenir des charges pulvérulentes, en particulier des argiles du type montmorillonite, l'hectorite ou la bentonite pour les produits de nettoyage ou d'autres charges telles que les silices ou les poudres de silicone (Tospearl) ou des polyamides (nylon) ou la poudre de polyméthacrylate de méthyle (Micropearl) pour obtenir des effets optiques.

On peut encore citer diverses charges de couleur blanche utilisées habituellement en cosmétologie, comme par exemple le talc, le kaolin, les poudres de teflon, de polyéthylène, de poly-bêta-alanine réticulée, etc...

Pour préparer les compositions de l'invention, on dissout les ingrédients solubles, ou on disperse les agents actifs insolubles, dans le milieu liquide aqueux, puis on ajoute le ou les agents gélifiants. On introduit ensuite les microsphères creuses expansées. Les charges peuvent être ajoutées avant ou après l'introduction du gélifiant. Le procédé ne nécessite ni une agitation violente ni un apport de chaleur si tous les composés sont solubles à température ambiante. On pourra ainsi utiliser sans problèmes des gels sensibles au cisaillement et des ingrédients sensibles à la température.

Parmi les compositions (ou supports) de l'invention, celles ayant de bonnes qualités d'onctuosité sont notamment celles qui contiennent de 0,1 à 2%, et en particulier de 0,5 à 1% en poids de microsphères creuses expansées par rapport au poids total de la composition, lesdites microsphères ayant des dimensions de 5-50µm et en particulier de 10-30µm. De telles compositions constituent notamment, sous la forme de gels onctueux, des produits de soins pour le visage (y compris des masques) ou pour le corps, des gels solaires, des produits après-rasage, des produits de maquillage du type fond-de-teint, blush ou fard à paupières, ou des produits de démaquillage.

Les compositions constituant un produit de nettoyage de la peau sous forme de masque pour le visage contiennent par exemple de 2 à 5% en poids de microsphères creuses ayant des dimensions de 10 à 50µm.

Les compositions de gommage pour la peau (scrubs) contiennent par exemple de 0,5 à 5% en poids de microsphères creuses expansées, lesdites microsphères ayant des dimensions moyennes de 80 à 250µm et en particulier de 100 à 250µm.

Les compositions dermopharmaceutiques selon l'invention sont des compositions pharmaceutiques appliquées par voie locale sur la peau ou les muqueuses.

Ces compositions peuvent contenir au moins un agent actif destiné notamment au traitement ou à la prévention des affections cutanées, y compris l'acné, les mycoses, les dermites séborrhéiques, l'eczéma, la rosacée, les héliodermatoses et le vieillissement cutané, ou encore les affections du cuir chevelu.

Parmi les agents actifs, on peut citer à titre d'exemple :
- les agents modulant la différenciation et/ou la prolifération et/ou la pigmentation cutanée tels que les composés dont l'action est médiée par les récepteurs nucléaires de la super-famille des stéroïdes/hormones thyroïdiennes, en particulier l'acide rétinoïque, ses isomères et ses dérivés par exemple le rétinol ou ses esters ainsi que des composés de synthèse analogues, par exemple l'acide 6-[3-(1-adamantyl)-4-méthoxyphényl] -2-naphtoïque ; la vitamine D ou ses dérivés tels que la 1,25-dihydroxy vitamine D₃ ou le calcipotriol ; les estrogènes tels que l'estradiol ;
- les agents antibiotiques tels que le phosphate de Clindamycine, l'érythromycine ou les antibiotiques de la classe des tétracyclines, par exemple la minocycline ;
- les antibactériens, en particulier le peroxyde de benzoyle ;
- les antimicrobiens, en particulier le métronidazole ;
- les antifongiques, en particulier les composés appartenant à la famille des imidazoles, tels que l'econazole ou l'omoconazole ou leurs sels, les composés polyènes, tels que l'amphotericine B ou les composés de la famille des allylamines, tels que la terbinafine ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène ou le diclofénac et leurs sels ;
- les agents antiprurigineux, tels que la capsaïcine ou les sels de lithium ;
- les agents analgésiques ;
- les agents antiviraux, tels que l'acyclovir ;
- les bloqueurs de canaux ioniques, tels que le minoxidil et ses dérivés ;
- des agents kératolytiques tels que les acides alpha-hydroxy- ou bêta-céto-carboxyliques, leurs sels, amides ou esters et plus particulièrement les acides alpha-hydroxylés, tels l'acide glycolique ou l'acide citrique ;
- les anti-radicaux libres, tels que l'alpha-tocophérol ou ses esters, les superoxyde dismutases ou certains chélatants des métaux.

La concentration utilisée dans les compositions dermopharmaceutiques dépend de la nature de l'agent actif ; elle est généralement comprise entre 0,001 % et 30 % en poids de la composition.

L'invention a également pour objet l'utilisation de microsphères creuses telles que définies précédemment comme additifs destinés à améliorer la facilité et la douceur d'application d'un gel cosmétique ou dermopharmaceutique aqueux exempt de matières grasses.

L'invention a également pour objet un procédé de traitement cosmétique caractérisé par le fait que l'on applique sur la peau une composition cosmétique telle que définie précédemment.

Les compositions dermopharmaceutiques de l'invention peuvent être utilisées notamment dans un procédé de traitement thérapeutique comprenant le traitement ou la prévention des affections cutanées y compris les héliodermatoses et le vieillissement cutané, ou encore les affections du cuir chevelu.

Les exemples suivants illustrent l'invention.

Dans ces exemples, les pourcentages sont en poids.

### EXEMPLE 1 : GEL POUR LA PEAU

| | |
|---|---|
| - Expancel EL 23 (KEMANORD) | 1% |
| - Carbopol 954 Goodrich (Carbomer) | 1,5% |
| - Polyvinylpyrrolidone | 1% |
| - Polyéthylèneglycol | 8,4% |
| - Triéthanolamine | 2,5% |
| - Conservateur | 0,5% |
| - Eau | qsp 100% |

Ce gel est préparé de la façon suivante : à l'eau contenant le conservateur on ajoute progressivement sous agitation le Carbopol, la polyvinylpyrrolidone et le polyéthylèneglycol; on neutralise par la triéthanolamine, puis on introduit l'Expancel à température ambiante.
- Résultat :: On obtient un gel de soin très doux, onctueux, non gras et non collant.

### Caractéristiques d'Expancel EL 23 :

| | |
|---|---|
| - Granulométrie moyenne | 18 µm |
| - Densité | 69,4 kg/m3 |
| - Gaz interne | isobutane |

### EXEMPLE 2 : BLUSH

De façon analogue, on a préparé un gel de composition suivante :

| | |
|---|---|
| - Expancel EL 23 (KEMANORD) | 1% |
| - Carbopol 954 Goodrich (Carbomer) | 1,50% |
| - Polyvinylpyrrolidone | 1% |
| - Polyéthylèneglycol | 8,40% |
| - Triéthanolamine | 2,50% |
| - Conservateur | 0,50% |
| - Colorant rouge soluble dans l'eau (sel disodique de ponceau) | 0,18% |
| - Colorant noir soluble dans l'eau (sel disodique du vert d'alizarine) | 0,22% |
| - Eau | qsp 100% |

- Resultats :: On obtient un blush sous la forme d'un gel onctueux, de couleur rouge rosé, facile à appliquer, doux. Le maquillage obtenu est très naturel et transparent.

### EXEMPLE 3 : FOND DE TEINT

| | |
|---|---|
| - Expancel EL 23 (KEMANORD) | 1% |
| - Carbopol 954 Goodrich (Carbomer) | 1,488% |
| - Polyvinylpyrrolidone | 0,99% |
| - Polyéthylèneglycol | 8,40% |
| - Triéthanolamine | 2,475% |
| - Glycérine | 2% |
| - Conservateur | 0,30% |
| - Colorant jaune soluble dans l'eau (jaune de quinoléine) | 0,07% |
| - Colorant rouge soluble dans l'eau (sel disodique de ponceau) | 0,09% |
| - Colorant noir soluble dans l'eau (sel disodique du vert d'alizarine) | 0,04% |
| - Eau | qsp 100% |

- Résultat :: On obtient un fond de teint sous forme de gel de couleur caramel, s'étalant très facilement, absolument non gras mais restant cependant onctueux et doux. Le maquillage est très naturel et de bonne tenue.

### EXEMPLE 4: SCRUB (Produit de nettoyage)

| | |
|---|---|
| - Expancel EL 16 (KEMANORD) | 1% |
| - Carbopol 954 Goodrich (Carbomer) | 2% |
| - Triéthanolamine | 2,50% |
| - Conservateur | 0,50% |
| - Glycérine | 3% |
| - Eau | qsp 100% |

- Résultat :: On obtient un produit de gommage doux pour la peau. On l'applique en massage sur la peau, puis on élimine le restant de la formulation par rinçage à l'eau. Il reste une peau très douce.

### Caractéristiques d'Expancel EL 16 :

| | |
|---|---|
| - Granulométrie moyenne | 90 µm |
| - Densité | 28,8 kg/m3 |
| - Gaz interne | isopentane |

### EXEMPLE 5 : GEL DE NETTOYAGE POUR LA PEAU

| | |
|---|---|
| - Expancel EL 3 (KEMANORD) | 1,5% |
| - Carbopol 954 GOODRICH (Carbomer) | 0,95% |
| - Triéthanolamine | 1% |
| - Butylèneglycol | 5% |
| - Carrageenan | 0,50% |
| - Dodécanediol polyglycérolé* | 2,50% |
| - Conservateur | 3% |
| - Eau | qsp 100% |

| | |
|---|---|
| *Dodécanediol polyglycérolé : produit obtenu par greffage de 3 moles de glycérol sur le dodécanediol ; voir FR.2.091.516. | |

- Résultat :: On obtient un gel de nettoyage très doux contenant des microbilles de grosse taille, visibles à l'oeil nu, ayant une action de massage et de nettoyage pendant l'application. On rince à l'eau comme pour un savon.

### Caractéristiques d'Expancel EL 3 :

| | |
|---|---|
| - Granulométrie moyenne | 87 µm |
| - Densité | 21 kg/m3 |
| - Gaz interne | isopentane |

### EXEMPLE 6 : MASQUE (produit de nettoyage pour le visage)

| | |
|---|---|
| - Expancel EL 4 (KEMANORD) | 5% |
| - Polyvinylpyrrolidone | 2,50% |
| - Polyglycérine 500 (SOLVAY) | 5% |
| - Conservateur | 3% |
| - Eau | qsp 100% |

- Résultat :: On obtient un produit épais d'aspect crémeux, que l'on étale sur la peau en couche épaisse et qu'on laisse sécher pendant 10 minutes. Le produit adsorbe les corps gras à la surface de la peau sans sensation de tiraillement. Il s'élimine très facilement à l'eau en laissant la peau très douce.

### Caractéristiques d'EXPANCEL EL 4 :

| | |
|---|---|
| - Granulométrie moyenne | 17 µm |
| - Densité | 115 kg/m3 |
| - Gaz interne | isobutane |

### EXEMPLE 7 : GEL SOLAIRE AQUEUX

| | |
|---|---|
| - Hydroxypropyl ether de cellulose* (PM : 300.000) | 3% |
| - Acide B-B' camphosulfonique (1-4 divinylbenzene) en solution aqueuse à 33% | 6,06% |
| - Triéthanolamine à 99% | 1,2 % |
| - Microsphères expansées (Expancel EL 23) | 0,5 % |
| - Eau déminéralisée stérilisée | qsp 100% |

| | |
|---|---|
| *dénomination commerciale KLUCEL G (Hercules) | |

Ce gel est frais et doux à l'application et protège du rayonnement solaire.

### EXEMPLE 8 : GEL APRES RASAGE

| | |
|---|---|
| - Copolymère acide acrylique/méthacrylate de stéaryle | 0,05% |
| - Polyéthylèneglycol 800 | 2,50% |
| - Glycérine | 1,50% |
| - Ethanol | 38% |
| - Allantoïne | 0,05% |
| - Menthol | 0,02% |
| - Triéthanolamine | 0,75% |
| - Conservateur | 0,01% |
| - Expancel EL 23 (KEMANORD) | 0,75% |
| - Eau | qsp 100% |

Ce gel est frais, doux et apaisant.

### EXEMPLE 9:

De façon analogue, on a préparé un scrub moussant dermopharmaceutique pour peaux acnéiques, de composition suivante (% en poids) :

| | |
|---|---|
| - Peroxyde de benzoyle | 5,00 |
| - Carbopol 940 (Goodrich) | 1,00 |
| - Glycérol | 5,00 |
| - Expancel EL 3 | 2,00 |
| - Butylèneglycol | 5,00 |
| - Dodécanediol polyglycérolé | 2,50 |
| - Hydroxyde de sodium | qsp pH 6 |
| - Eau purifiée | qsp 100,00 |

- Résultat :: On obtient un gel nettoyant adapté au traitement des peaux acnéiques. Ce gel est utilisé en application quotidienne avec un léger massage, suivie d'un rinçage à l'eau.

### EXEMPLE 10 :

De façon analogue, on a préparé un gel dermopharmaceutique antiacnéique, de composition suivante (% en poids) :

| | |
|---|---|
| - Acide 6-[3(1-adamantyl)-4-méthoxyphényl] 2-naphtoïque | 0,10 |
| - Carbopol 940 (Goodrich) | 1,10 |
| - Propylèneglycol | 4,00 |
| - Expancel EL 23 | 1,00 |
| - Parahydroxybenzoate de méthyle | 0,10 |
| - Phénoxyéthanol | 0,25 |
| - Hydroxyde de sodium | qsp pH 5 |
| - Eau | qsp 100,00 |

- Résultat :: On obtient un gel non gras et non collant, actif en application quotidienne dans le traitement de l'acné et dans le traitement ou la prévention des signes du vieillissement cutané.

### EXEMPLE 11 :

De façon analogue, on a préparé un gel dermopharmaceutique antifongique de composition suivante (% en poids) :

| | |
|---|---|
| - Omoconazole nitrate | 1,00 |
| - Carbopol 954 (Goodrich) | 1,50 |
| - Propylène glycol | 8,40 |
| - Expancel EL 23 | 0,50 |
| - Polyvinylpyrrolidone | 1,00 |
| - Parahydroxybenzoate de méthyle | 0,20 |
| - Hydroxyde de sodium | qsp pH 5 |
| - Eau | qsp 100,00 |

- Résultat :: On obtient un gel actif en application quotidienne dans le traitement du Pithyriasis Versicolor et de la dermite séborrhéique.

### EXEMPLE 12 :

De façon analogue, on a préparé un gel dermopharmaceutique pour le traiement de la rosacée de composition suivante (% en poids) :

| | |
|---|---|
| - Métronidazole | 0,75 |
| - Carbopol 940 (Goodrich) | 0,60 |
| - Polyéthylène glycol | 3,00 |
| - Expancel EL 23 | 1,50 |
| - Parahydroxybenzoate de méthyle | 0,10 |
| - Hydroxyde de sodium | qsp pH 5 |
| - Eau | qsp 100,00 |

- Résultat :: On obtient un gel non gras, non collant et légèrement couvrant, actif en application quotidienne sur le visage dans le traitement de la rosacée.

### EXEMPLE 13 :

De façon analogue, on a préparé un gel dermopharmaceutique antiinflammatoire de composition suivante (% en poids) :

| | |
|---|---|
| - Propionate de clobétasol | 0,05 |
| - Carbopol 940 (Goodrich) | 0,60 |
| - Propylène glycol | 20,00 |
| - Expancel EL 23 | 0,50 |
| - Parahydroxybenzoate de méthyle | 0,20 |
| - Hydroxyde de sodium | qsp pH 5 |
| - Eau | qsp 100,00 |

- Résultat :: On obtient un gel actif en application quotidienne dans le traitement des affections inflammatoires telles que l'eczéma atopique.

Les exemples comparatifs suivants montrent que les résultats intéressants procurés par l'addition aux gels aqueux de microsphères creuses expansées ne sont pas obtenus avec d'autres microsphères.

### EXEMPLE COMPARATIF N°1

### Gel contenant des microbilles de résine de silicone

Ce gel a la composition suivante (% en poids) :

| | |
|---|---|
| - Sphères de silicone : TOSPEARL 108* (TOSHIBA) | 5% |
| - Carbopol 954 GOODRICH (Carbomer) | 1,5% |
| - Polyvinylpyrrolidone | 1% |
| - Polyéthylèneglycol | 8,40% |
| - Triéthanolamine | 2,50% |
| - Conservateur | 0,30% |
| - Eau | 81,30% |

| | |
|---|---|
| *TOSPEARL 108 (marque commerciale) : cette charge, de très fine granulométrie est réputée pour son apport de douceur, dans les milieux aqueux cosmétiques. | |

- Résultat :: A l'application, on constate une diminution rapide de la facilité d'étalement et un effet poisseux et sec. Le produit finit par pelucher lorsqu'on continue le massage.
- Remarque :: Si l'on prépare une formule analogue avec 10% de Tospearl 108, on obtient un résultat identique.

### EXEMPLE COMPARATIF N°2

### Gel contenant des sphères microporeuses de polyméthacrylate de méthyle

| | |
|---|---|
| - Sphères de Micropearl M* (MATSUMOTO) | 5% |
| - Carbopol 954 (GOODRICH) | 1,5% |
| - Polyvinylpyrrolidone | 1% |
| - Polyéthylèneglycol | 8,40% |
| - Triéthanolamine | 2,50% |
| - Conservateur | 0,30% |
| - Eau | 81,30% |

| | |
|---|---|
| *Micropearl M : poudre sphérique ultra-fine microporeuse à cavités ouvertes, développée spécialement pour les applications cosmétiques, au toucher très doux pour la peau ; taille = 10 µm | |

- Résultats :: - Gel de bel aspect,
- Poisseux à l'application,
- Effet très sec,
- Pelucheux.
- Remarque :: On obtient un résultat identique avec 10% de Micropearl M.

### EXEMPLE COMPARATIF N°3

### Gel aux microbilles de silice avec une cavité SB 700 - Silica Beads

| | |
|---|---|
| - SB 700* | 5% |
| - Carbopol 954 GOODRICH (Carbomer) | 1,5% |
| - Polyvinylpyrrolidone | 1% |
| - Polyéthylèneglycol | 8,40% |
| - Triéthanolamine | 2,50% |
| - Conservateur | 0,30% |
| - Eau | 81,30% |

| | |
|---|---|
| *SB 700 : poudre d'origine minérale microporeuse, hydrophile, de taille de 1 à 17 µm (MAPRECOS) | |

- Résultats :: Pendant et après l'application, le toucher de la peau n'est pas agréable.

Ces exemples comparatifs démontrent que l'on ne peut pas obtenir, même avec d'autres microsphères creuses, les effets cosmétiques conférés aux gels aqueux par les microsphères creuses expansées en matériau thermoplastique, qui ont la particularité d'apporter beaucoup de douceur d'application et de confort.

## Revendications

1. Composition cosmétique ou dermopharmaceutique, ou support de composition cosmétique ou dermopharmaceutique, sous forme de gel modifié, caractérisé(e) par le fait que ladite composition ou ledit support comprend des microsphères creuses dispersées dans un gel aqueux, que lesdites microsphères creuses sont des microsphères expansées, en matériau thermoplastique ayant une masse spécifique de 15 à 200 kg/m3, que ladite composition ou ledit support contient de 0,1 à 10% en poids desdites microsphères par rapport au poids total de la composition ou du support, et que ladite composition ou ledit support est exempt(e) de matières grasses.

2. Composition ou support selon la revendication 1, caractérisé(e) par le fait que lesdites microsphères creuses ont des dimensions moyennes de particules pouvant varier de 5 à 250 µm, et en particulier de 10 à 150 µm.

3. Composition ou support selon l'une quelconque des revendications précédentes, caractérisé(e) par le fait que ledit gel comprend au moins un agent gélifiant dans un véhicule liquide aqueux, ledit agent gélifiant étant choisi parmi les polymères hydrosolubles ou donnant, dans l'eau, des solutions colloïdales.

4. Composition ou support selon la revendication précédente, caractérisé(e) par le fait que ledit agent gélifiant est choisi parmi les polymères ou copolymères d'acides organiques carboxyliques insaturés ou d'esters insaturés, les dérivés polysaccharidiques, les gommes, les silicates colloïdaux, les polyéthylèneglycols (PEG) et leurs dérivés, les polyvinylpyrrolidones et leurs dérivés, et les gels de silice hydrophiles.

5. Composition ou support selon la revendication 3 ou 4, caractérisé(e) par le fait que ledit agent gélifiant est choisi parmi les polymères ou copolymères acryliques et/ou méthacryliques, les polymères carboxyvinyliques, les acrylates ou méthacrylates de polyglycéryle, les dérivés de cellulose ou d'amidon, les dérivés de chitine, les alginates, l'acide hyaluronique et ses sels, les chondroïtine sulfates, les gommes de xanthane, de gellane, de Rhamsan, de karoya, ou de guar, la farine de caroube, les silicates d'aluminium et de magnésium colloïdaux de type montmorillonite, les polyacrylamides et leurs dérivés, les polyacrylonitriles.

6. Composition ou support selon l'une quelconque des revendications 3 à 5, caractérisé(e) par le fait que ledit agent gélifiant est présent en quantité suffisante pour conférer à ladite composition la viscosité souhaitée pour ladite composition ou ledit support.

7. Composition ou support selon la revendication précédente, caractérisé(e) par le fait que ladite viscosité peut aller de 3 à 200 poises (soit 0,3 à 20 Pa.s).

8. Composition ou support selon l'une quelconque des revendications précédentes, sous forme de gel onctueux, caractérisé(e) par le fait qu'elle contient lesdites microsphères creuses à raison de 0,1 à 2% en poids par rapport au poids total de la composition, et que lesdites microsphères ont des dimensions de 5-50µm et en particulier de 10-30µm.

9. Composition cosmétique selon l'une quelconque des revendications 1 à 7, caractérisé(e) par le fait qu'elle contient de 2 à 5% en poids par rapport au poids total de la composition, desdites microsphères, lesdites microsphères ayant des dimensions de 10 à 50 µm, ladite composition constituant un produit de nettoyage de la peau sous forme de masque pour le visage.

10. Composition selon l'une quelconque des revendications 1 à 7, caractérisée par le fait qu'elle contient de 0,5 à 5% en poids desdites microsphères, et que lesdites microsphères ont des dimensions moyennes de 80 à 250 µm, et en particulier de 100 à 250 µm, et que ladite composition constitue un produit de gommage pour la peau.

11. Utilisation de microsphères creuses telles que définies dans l'une quelconque des revendications 1, 2, 8, 9 et 10, comme additifs destinés à améliorer la facilité et la douceur d'application d'un gel cosmétique ou dermopharmaceutique aqueux exempt de matières grasses.

12. Procédé de traitement cosmétique, caractérisé par le fait que l'on applique sur la peau une composition cosmétique telle que définie dans l'une quelconque des revendications 1 à 10.

## Claims

1. Cosmetic or dermopharmaceutical composition, or a vehicle for a cosmetic or dermopharmaceutical composition, in the form of a modified gel, characterized in that the said composition or said vehicle comprises hollow microspheres dispersed in an aqueous gel, in that the said hollow microspheres are expanded microspheres made of thermoplastic material having a density of 15 to 200 kg/m³, in that the said composition or said vehicle contains 0.1 to 10 % by weight of the said microspheres relative to the total weight of the composition or vehicle, and in that the said composition or said vehicle is free from fats.

2. Composition or vehicle according to Claim 1, characterized in that the said hollow microspheres have average particle sizes which can vary from 5 to 250 µm, and especially from 10 to 150 µm.

3. Composition or vehicle according to either of the preceding claims, characterized in that the said gel comprises at least one gelling agent in an aqueous liquid vehicle, the said gelling agent being chosen from polymers which are water-soluble or which give colloidal solutions in water.

4. Composition or vehicle according to the preceding claim, characterized in that the said gelling agent is chosen from polymers or copolymers of unsaturated organic carboxylic acids or of unsaturated esters, polysaccharide derivatives, gums, colloidal silicates, polyethylene glycols (PEG) and their derivatives, polyvinylpyrrolidones and their derivatives and hydrophilic silica gels.

5. Composition or vehicle according to Claim 3 or 4, characterized in that the said gelling agent is chosen from acrylic and/or methacrylic polymers or copolymers, vinylcarboxylic polymers, polyglyceryl acrylates or methacrylates, cellulose or starch derivatives, chitin derivatives, alginates, hyaluronic acid and its salts, chondroitin sulphates, xanthan, gellan, Rhamsan, karaya or guar gum, carob flour, colloidal aluminium magnesium silicates of the montmorillonite type, polyacrylamides and their derivatives and polyacrylonitriles.

6. Composition or vehicle according to any one of Claims 3 to 5, characterized in that the said gelling agent is present in a sufficient amount to endow the said composition with the viscosity which is desired for the said composition or said vehicle.

7. Composition or vehicle according to the preceding claim, characterized in that the said viscosity can range from 3 to 200 poises (equivalent to 0.3 to 20 Pa.s)

8. Composition or vehicle according to any one of the preceding claims, in the form of a creamy gel, characterized in that it contains the said hollow microspheres in the proportion of 0.1 to 2 % by weight relative to the total weight of the composition, and in that the said microspheres are 5-50 µm, and especially 10-30 µm, in size.

9. Cosmetic composition according to any one of Claims 1 to 7, characterized in that it contains 2 to 5 % by weight, relative to the total weight of the composition, of the said microspheres, the said microspheres being 10 to 50 µm in size, the said composition constituting a skin cleansing product in the form of a face mask.

10. Composition according to any one of Claims 1 to 7, characterized in that it contains 0.5 to 5 % by weight of the said microspheres, and in that the said microspheres have average sizes of 80 to 250 µm, and especially 100 to 250 µm, and in that the said composition constitutes an exfoliating product for the skin.

11. Use of hollow microspheres as are defined in any one of Claims 1, 2, 8, 9 and 10, as additives intended for improving the ease and gentleness of application of an aqueous cosmetic or dermopharmaceutical gel free from fats.

12. Cosmetic treatment process, characterized in that a cosmetic composition as defined in any one of Claims 1 to 10 is applied to the skin.

## Patentansprüche

1. Kosmetische oder dermopharmazeutische Zubereitung, oder kosmetische oder dermopharmazeutische Zubereitungsgrundlage, in Form eines modifizierten Gels, dadurch gekennzeichnet, daß die Zubereitung oder Grundlage in wäßrigem Gel dispergierte Mikrohohlkügelchen enthält, daß die Mikrohohlkügelchen aufgeschäumte Mikrokügelchen aus thermoplastischem Material mit einer spezifischen Masse von 15 bis 200 kg/m³ sind, daß die Zubereitung oder die Grundlage 0,1 bis 10 Gew.-% an Mikrokügelchen, bezogen auf das Gesamtgewicht der Zubereitung oder Grundlage, enthält, und daß die Zubereitung oder Grundlage frei von Fettstoffen ist.

2. Zubereitung oder Grundlage nach Anspruch 1, dadurch gekennzeichnet, daß die Mikrohohlkügelchen eine mittlere Teilchengröße von 5 bis 250 µm, insbesondere von 10 bis 150 µm aufweisen.

3. Zubereitung oder Grundlage nach einem der vorhergehenden Ansprüche, dadurch gekennzeichnet, daß das Gel zumindest ein in einem flüssigen, wäßrigen Träger gelierendes Mittel eilthält, wobei das Geliermittel aus wasserlöslichen oder in Wasser kolloidale Lösungen erzeugende Polymeren ausgewählt ist.

4. Zubereitung oder Grundlage nach dem vorhergehenden Anspruch, dadurch gekennzeichnet, daß das Geliermittel aus Polymeren oder Copolymeren organischer ungesättiger Carbonsäuren oder ungesättiger Ester, aus Polysaccharidderivaten, Gummen, kolloidalen Silicaten, Polyethylenglykolen (PEG) und ihren Derivaten, Polyvinylpyrrolidonen und ihren Derivaten und aus hydrophilen Kieselsäure-Gelen ausgewählt ist.

5. Zubereitung oder Grundlage nach Anspruch 3 oder 4, dadurch gekennzeichnet, daß das Geliermittel aus Acryl- und/oder Methacrylpolymeren und/oder -copolymeren, Carboxyvinylpolymeren, Polyglycerinacrylaten oder - methacrylaten, Zellulose- oder Stärkederivaten, Chitinderivaten, Alginaten, Hyaluronsäure und deren Salzen, Chondroitinsulfaten, Xanthan-, Gellan-, Rhamsan-, Karoya- oder Guargummen, Johannisbrotkernmehl, kolloidalen Aluminium- und Magnesiumsilicaten vom Typ Montmorillonit, Polyacrylamiden und deren Derivaten, und aus Polyacrylnitrilen ausgewählt ist.

6. Zubereitung oder Grundlage nach einem der Ansprüche 3 bis 5, dadurch gekennzeichnet, daß das Geliermittel in ausreichender Menge vorhanden ist, um der genannten Zubereitung die für die Zubereitung oder Grundlage gewünschte Viskosität zu verleihen.

7. Zubereitung oder Grundlage nach dem vorhergehenden Anspruch, dadurch gekennzeichnet, daß die genannte Viskosität 3 bis 200 Poise (d. h. 0,3 bis 20 Pa · s) beträgt.

8. Zubereitung oder Grundlage nach einem der vorhergehenden Ansprüche, in Form von salbenartigem Gel, dadurch gekennzeichnet, daß sie Mikrohohlkügelchen in einem Verhältnis von 0,1 bis 2 Gew.-%, bezogen auf das Gesamtgewicht der Zubereitung enthält, und daß die genannten Mikrokügelchen eine Größe von 5 - 50 µm, insbesondere von 10 - 30 µm aufweisen.

9. Kosmetische Zubereitung nach einem der Ansprüche 1 bis 7, dadurch gekennzeichnet, daß sie 2 bis 5 Gew.-% Mikrokügelchen, bezogen auf das Gesamtgewicht der Zubereitung, enthält, wobei die Mikrokügelchen eine Größe von 10 bis 50 µm aufweisen, und wobei die Zubereitung ein Reinigungsmittel für die Haut in Form einer Gesichtsmaske darstellt.

10. Zubereitung nach einem der Ansprüche 1 bis 7, dadurch gekennzeichnet, daß sie 0,5 bis 5 Gew.-% an Mikrokügelchen enthält, und daß die Mikrokügelchen eine mittlere Größe von 80 bis 250 µm, insbesondere von 100 bis 250 µm aufweisen, und daß die Zubereitung ein Gummierungsmittel für die Haut darstellt.

11. Verwendung von Mikrohohlkügelchen wie in einem der Ansprüche 1, 2, 8, 9 und 10 definiert, als Zusätze zur Verbesserung der Anwendungsfreundlichkeit und der Geschmeidigkeit eines kosmetischen oder dermopharmazeutischen, wäßrigen Gels ohne Fettstoffe.

12. Verfahren zur kosmetischen Behandlung, dadurch gekennzeichnet, daß eine wie nach einem der Ansprüche 1 bis 10 definierte kosmetische Zubereitung auf die Haut aufgetragen wird.
